# EUROPEAN PATENT SPECIFICATION

(11) **EP 4 622 705 B1**
(45) Date of publication and mention of the grant of the patent: **21.10.2026**
(21) Application number: 24817042.5
(22) Date of filing: 05.11.2024
(51) Int. Cl.: A61N 1/32, A61N 1/36

(54) **APPARATUS FOR ELECTRIC FIELD STIMULATION OF A BIOLOGICAL TISSUE REGION**
VORRICHTUNG ZUR STIMULATION EINER BIOLOGISCHEN GEWEBEREGION MITTELS EINES ELEKTRISCHEN FELDES
APPAREIL DE STIMULATION PAR CHAMP ÉLECTRIQUE D'UNE RÉGION DE TISSU BIOLOGIQUE

(30) Priority: 06.11.2023 EP 23208090
(43) Date of publication of application: 01.10.2025
(73) Proprietor: Foundation for Research on Information Technologies in Society, 8004 Zürich (CH)
(72) Inventor: CAPSTICK, Myles, 8004 Zürich (CH)
(74) Representative: Schmauder & Partner AG Patent- & Markenanwälte VSP
(86) International application number: PCT/EP2024/081230
(87) International publication number: WO 2025/099015

(56) References cited:
- WO-A1-2016/057855
- WO-A1-2021/044168
- US-A1- 2018 185 649

## Description

The present invention generally relates to an apparatus for electric field stimulation of a biological tissue region, particularly in a living human body.

### Field of the invention

Electric field stimulation of a human or animal organism is generally understood as a method in which applied, generally time-varying electric fields are used to cause a desired reaction in the organism. The method relies on the possibility for an applied electric field to generate an electrophysiological response in biological tissues, for example in the generation of neural action potentials or the modulation of sub-threshold dynamics. Examples of such excitable cells are neurons, muscle cells and some endocrine cells. One known method of electric field stimulation, which is called interferential stimulation, relies on generating a desired spatio-temporal electric field distribution in the biological tissue region of interest. Apparatuses and methods implementing interferential stimulation are disclosed, e.g., in WO 2016/057855 A1 (MIT) and WO 2021/044168 A1 (ICI). WO 2016/057855 A1 (MIT) is based on creating a first electric field between electrodes in a first pair of electrodes, and creating a second electric field between electrodes in a second pair of electrodes, such that, firstly, when the two pairs of electrodes are electrically connected to a brain, the first and second electric fields constructively and destructively interfere with each other over time to create an amplitude-modulated waveform, and, secondly, the locations of largest envelope amplitude of the amplitude-modulated waveform can be remote from the electrode. Example applications include stimulation of deep brain regions while avoiding activation of overlaying regions.

WO 2021/044168 A1 (ICI) also relies on using two alternating electric fields which are directed through a biological tissue, the two alternating electric fields having, respectively, a first frequency and a second frequency. The two alternating electric fields provide a combined field in the biological tissue. By varying at least one of the two frequencies, the combined field provides a pulsed interferential stimulation signal.

Terasawa et al., IEEE Transactions on Biomedical Engineering, Vol. 69, No. 1, January 2022, describe a method of temporal control of interferential neural stimulation using two carriers via phase modulation. As in related work, Terasawa et al. apply a sinusoidal current to each one of two pairs of electrodes, thereby causing interference. But instead of using slightly different frequencies, they use two sinusoidal currents with identical frequencies. Temporal control of the resulting envelope is achieved by linearly changing the phase of one sinusoid with respect to the other from π to 0 at the start of the envelope pulse then from 0 back to π at the end of the pulse. Hence the shape of the pulse transitions is always the same, though not the overall pulse length or rate of change of envelope amplitude with respect to pulse repetition rate.

### Summary of the invention

The invention is defined by the independent claim 1. It is the principal object of the present invention to overcome the limitations and disadvantages of currently known apparatuses.

Therefore, according to the present invention, there is provided an apparatus for electric field stimulation of a biological tissue region, the apparatus comprising:
a) a plurality N of at least two electrode pairs, each electrode being formed for application on a surface segment of, or implanted into, said biological tissue region, and
b) for each electrode pair, a respective electric driving source for generating a respective electric field between electrodes of said pair.

In the present context, the term "biological issue region" shall be understood as a region of biological tissue, including, without limitation, a region of biological tissue of an organism, particularly of a living organism, wherein the organism can be an animal or a hu-man, but also including a region of biological tissue constituting a sample in an in-vitro setting.

Each electric driving source is configured to generate a respective sinusoidally time dependent electric field having a respective carrier frequency and a respective amplitude, whereby a resultant time dependent electric field having a phase modulation frequency is formed in a region wherein respective electric field vectors spatially overlap.

The apparatus comprises control means for operating all of said electric driving sources in such manner that
- all of said respective frequencies are identical to one preselected carrier frequency f₀, the carrier frequency f₀ being selected from 500 Hz to 1 MHz,
- each one of said respective sinusoidally time dependent electric fields has a defined normalized time-dependent phase difference for one cycle (φᵢ(t), with i=1 to N) with respect to a reference phase (φ₀ = 0), wherein ${φ}_{i} \left(t\right) = \left(i-1\right) ⋅ φ \left(t\right)$
- where *φ*(*t*) varies from -2π/N to +2π/N
- and, if the phase modulation frequency is desired to be fₘ, then the time-dependent phase difference is given by
${φ}_{i} \left(t\right) = \left(i-1\right) ⋅ φ \left(t\right) ⋅ {f}_{m} .$

The control means for operating the electric driving sources can be configured such that the carrier frequency f₀ can be selected continuously within the respective overall carrier frequency range and the phase modulation frequency fₘ can be selected continuously from 0 to several hundred Hz. Alternatively, the control means can be configured to provide a discrete selection of f₀ and fₘ.

In a non-claimed method of electric field stimulation of biological tissue in a biological body by means of an apparatus as defined above, the method comprises carrying out at least one stimulation cycle comprising the steps of:
i) for the number N of said plurality of electrode pairs, determining a phase modulation function φ(t) in a normalized time range t = 0 to 1 corresponding to a chosen normalized envelope pulse shape function p(t) in said normalized time range;
ii) generating respective sinusoidally time dependent electric fields, each having a preselected time-dependent phase difference (φᵢ(t), with i=1 to N) with respect to a reference phase (φ₀ = 0), wherein: ${φ}_{i} \left(t\right) = \left(i-1\right) ⋅ φ \left(t\right)$ where *φ*(*t*) is a function of the required envelope shape and varies from -2π/N to +2π/N;
whereby the resultant electric field in a region wherein the respective aligned electric field vectors spatially overlap with equal amplitude has a temporal envelope shape corresponding to said normalized envelope pulse shape function p(t) in said normalized time range.

In the following, the term "carrier" will be used to compactly refer to each one of the various sinusoidally time dependent electric fields defined above.

That all of the above-mentioned respective frequencies are identical to one preselected carrier frequency f0 shall not be understood in a strict mathematical sense, but rather in the context of available frequency generator devices.

The present invention relies on using a time-dependent phase modulation scheme according to which
- the various carriers interacting with each other to form a resultant time dependent electric fields all have the same carrier frequency,
- each carrier is provided with a respective time-dependent phase,
- the time-dependent phases of the various carriers are set up in such a manner as to generate a chosen temporal envelope shape of the resultant overlapping time-dependent electric field vector components.

As will be understood, it is convenient to express the time-dependent phase of each carrier with respect to a common reference phase, which will also determine the - generally time dependent - phase difference between any pair of carriers.

According to the invention, the time-dependent phase difference (φᵢ(t), with i=1 to N) with respect to a reference phase (φ₀), to obtain an envelope shape repeating at frequency fₘ is set up to be ${φ}_{i} \left(t\right) = \left(i-1\right) ⋅ φ \left(t\right) ⋅ {f}_{m}$

Without loss of generality, the reference phase φ₀ can be set equal to zero.

For the purposes of the present invention, the carrier frequency is selected in the range of 500 Hz to 1 MHz. The lower frequency limit is chosen so as to be sufficiently high to avoid direct stimulation of the biological tissue. The higher frequency limit is chosen for reasons of design and practicality. The above-mentioned frequencies correspond to vacuum wavelengths in the order of 600 km and 300 m, respectively. Accordingly, for a region of interest in a biological body such as a human or animal patient, which will typically not exceed a size of 1 m, the phase of each carrier at a given instant of time is substantially constant across the entire region of interest. This means that the spatial intensity distribution of the resultant time-dependent electric field envelope in the region of interest is determined by the spatially overlapping distributions of the various carrier field vectors and by possible material inhomogeneities within the biological tissue. The interferential stimulation according to the present invention sets out to provide a desired temporal variation of the resultant electric field summation envelope within a target region. Advantageous embodiments are defined in the dependent claims and in the examples.

The apparatus of the present invention requires using a plurality N of at least two electrode pairs. However, in various embodiments the plurality N of electrode pairs is at least three (claim 2).

Advantageously (claim 3), the control means comprise:
i) means for inputting or selecting a pulse shape function p(t) in a normalized time range t = 0 to 1,
ii) means for determining said phase modulation function φ(t) in said normalized time range,
iii) means for generating said respective sinusoidally time dependent electric fields, each having a preselected time-dependent phase difference (φᵢ(t), with i=1 to N) with respect to a reference phase φ₀, wherein: ${φ}_{i} \left(t\right) = \left(i-1\right) ⋅ φ \left(t\right) ⋅ {f}_{m}$
whereby the resultant electric field in a region wherein the respective aligned electric field vectors spatially overlap has a temporal envelope shape corresponding to said normalized envelope pulse shape function p(t) in said normalized time range.

In one advantageous embodiment, (claim 4), the phase modulation function φ(t) is: $φ \left(t\right) = \frac{4}{N} ⋅ acos \left(p{\left(t\right)}^{\frac{1}{a \left(N\right)}}\right) ⋅ sign \left(\frac{d}{dt}acos \left(p{\left(t\right)}^{\frac{1}{a \left(N\right)}}\right)\right)$ wherein a(N) is an envelope correction factor.

In another advantageous embodiment, (claim 5), the phase modulation function φ(t) is: $φ \left(t\right) = \pm abs \left[\frac{4}{N}⋅acos \left(p{\left(t\right)}^{\frac{1}{a \left(N\right)}}\right)⋅sign\left(\frac{d}{dt}acos \left(p{\left(t\right)}^{\frac{1}{a \left(N\right)}}\right)\right)\right]$ wherein a(N) is an envelope correction factor.

It will be understood that in the above cases *φ*(*t*) varies from -2π/N to +2π/N.

In particular (claim 6), when the plurality of electrode pairs is larger than two, the envelope correction factor a(N) is $a \left(N\right) = 1 + 0.25 \left(1-{e}^{- \frac{N - 2}{1.356}}\right)$

This correction factor does not have to be applied, i.e. a(N) can be set to 1 (claim 7), some distortion of the envelope shape is acceptable in the given application. In certain embodiments (claim 8), said field generating means iii) are configured to carry out a temporally repetitive sequence of stimulation cycles. Accordingly, the method steps i) and ii) as defined above are carried out repetitively so as to form said temporal sequence of stimulation cycles. The number of repetitions will generally depend on the specific application.

In many embodiments, the stimulation cycles are identical.

According to another embodiment (claim 9), the apparatus comprises first means for inputting or selecting a first normalized envelope pulse shape function p1(t) in a first normalized time range and second means for inputting or selecting a second normalized envelope pulse shape function p2(t) in a second normalized time range that switches to follow directly after the first normalized time range upon receiving a command to change envelope shape, wherein said first and second normalized envelope pulse shapes are configured to smoothly switch from said first normalized envelope pulse shape function p1(t) to said second normalized envelope pulse shape function p2(t) synchronously for all electrode pairs of a plurality.

In particular (claim 10), the apparatus can be configured in such manner that phase modulation data is read out of a phase modulation memory block at a controlled rate so as to ensure both carrier and envelope phase continuity when modifying the phase modulation frequency (fₘ).

In a corresponding variant of the non-claimed method, a first normalized envelope pulse shape function p1(t) is chosen in a first normalized time range and a second normalized envelope pulse shape function p2(t) is chosen in a second normalized time range that follows after the first normalized time range, wherein said first and second normalized envelope pulse shapes are selected so as to smoothly switch from said first normalized envelope pulse shape function p1(t) to said second normalized envelope pulse shape function p2(t) synchronously for all electrode pairs of a plurality. In the course of such a temporal sequence, the interferential envelope repetition rate or frequency can be modified, for which purpose phase modulation data is read out of a phase modulation memory block at a controlled rate so as to ensure both carrier and envelope phase continuity when modifying the phase modulation frequency (fₘ). For this purpose, one can arrange to have two working data sets for modulation, with switching from one to the other only when the address counter resets to the first data element.

In another class of embodiments (claim 11), the interferential envelope repetition rate or frequency (fₘ) can be modified to synchronize the amplitude modulation envelope phase and frequency with an external input trigger pulse train. In these embodiments the rate of reading of the phase data φᵢ(t) is the quantity modified to achieve the carrier envelope phase lock.

Another class of embodiments (claim 12) aims at generating two different sham exposure states in a biological tissue of interest, in which no time varying interferential envelope modulation p(t) is present, wherein in the first sham exposure state the carrier frequency induced fields in the biological tissue minimize the total field magnitude and wherein in the second sham exposure state the carrier frequency induced fields in the biological tissue all are in phase.

Another class of embodiments (claim 13) exploits the combination of
a) the placement of each electrode pair of said plurality being chosen such that the generates respective electric field between each pair of said electrodes with a given direction in the target region, and
b) the time dependent phase difference between each pair of electrodes in the plurality being chosen such that the superposition of the fields results in a time dependent variation in polarization of from linear through elliptical and back to linear,
whereby appropriate alignment of fields within tissues having varying levels of excitability depending on the direction of the electric field vector experience a time dependent excitation of the tissue.

In yet another class of embodiments (claim 14), the time-dependent phase differences *φᵢ*(*t*) is such that the rate of change of electric field polarization is modified such that the product of the angular excitability and polarization angle result in a desired excitation as a function of time.

According to one embodiment of the apparatus (claim 15), the electric driving sources each comprise a digital synthesizer, with all digital synthesizers running from a common clock so as to be synchronizable to said common reference phase (φ₀).

According to another embodiment of the apparatus (claim 16), each one of the electric driving sources comprises an electric network that converts an output signal of a digital synthesizer to a corresponding differential electric voltage stimulus or current stimulus of a desired amplitude that excites an associated electrode pair.

Another embodiment (claim 17) allows to independently excite two different regions of the biological body or to enhance the excitation of a single region for example by having different dominant electric field vector directions. This is provided by the apparatus comprising
c) a further plurality (N') of at least two electrode pairs, each electrode being formed for application on a surface segment of, or implanted in, said biological body,
d) for each electrode pair of said further plurality, a respective further electric driving source for generating a respective electric field between electrodes of said pair,

wherein each further electric driving sources are configured to generate respective sinusoidally time dependent further electric field having a respective further carrier frequency and a respective further amplitude,
whereby a resultant time dependent further electric fields are formed in a region wherein said further respective electric field vectors spatially overlap.

In this embodiment, the apparatus comprises further control means for operating all of said further electric driving sources in such manner that
- all of said respective further carrier frequencies are identical to one preselected further carrier frequency (f₀'), the further carrier frequency (f₀') being selected from 500 Hz to 1 MHz,
- each one of said respective further sinusoidally time dependent electric fields has a preselected time-dependent further phase difference (φᵢ'(t), with i=1 to N') with respect to a further reference phase (φ₀'), wherein ${φ}_{i} ′ \left(t\right) = \left(i-1\right) ⋅ φ ′ \left(t\right)$
   - and, if the frequency of the phase modulation is desired to be fₘ, then the time-dependent further phase difference is given by ${φ}_{i} ′ \left(t\right) = \left(i-1\right) ⋅ φ ′ \left(t\right) ⋅ f {′}_{m}$
wherein said carrier frequency (f₀) and said further carrier frequency (f₀') are spaced apart by a frequency difference (Δf) which is at least 500 Hz. This ensures that the difference in frequency between all carriers is sufficiently high that there is no direct or interferential stimulation of the biological tissue. In this embodiment, a plurality of two or more substantially independent electrode pairs is implemented. In other words, the term "further" is to be understood in the sense of "at least one further".

The corresponding non-claimed method involves the following steps
i) for the number (N) of said plurality of electrode pairs, determining a phase modulation function φ(t) in a normalized time range t = 0 to 1 corresponding to a chosen normalized envelope pulse shape function p(t) in said normalized time range;
ii) generating respective sinusoidally time dependent electric fields, each having a preselected time-dependent phase difference (φᵢ(t), with i=1 to N) with respect to a reference phase (φ₀), wherein: ${φ}_{i} \left(t\right) = \left(i-1\right) ⋅ φ \left(t\right) ⋅ {f}_{m}$ where *φ*(*t*) is a function of the required envelope shape and varies from -2π/N to +2π/N
whereby the resultant electric field in a region wherein the respective aligned electric field vectors spatially overlap has a temporal envelope shape corresponding to said normalized envelope pulse shape function p(t) in said normalized time range;
and
i') for the number (N') of said further plurality of electrode pairs, determining a further phase modulation function φ'(t) in said normalized time range t = 0 to 1 corresponding to a chosen normalized further envelope pulse shape function p'(t) in said normalized time range;
ii') generating respective further sinusoidally time dependent electric fields, each having a preselected time-dependent phase difference (φ_{i'}'(t), with i=1 to N') with respect to a further reference phase (φ₀'), wherein: ${φ}_{i} ′ \left(t\right) = \left(i-1\right) ⋅ φ ′ \left(t\right) ⋅ f {′}_{m}$ where *φ'*(*t*) is a function of the required envelope shape and varies from -2π/N to +2π/N

whereby the resultant further electric field in a region wherein the respective aligned electric field vectors spatially overlap has a temporal envelope shape corresponding to said normalized further envelope pulse shape function p'(t) in said normalized time range;
wherein said carrier frequency (f₀) and said further carrier frequency (f₀') are spaced apart by a frequency difference (Δf) which is at least 500 Hz.

In one class of embodiments (claim 18), each plurality of carriers is phase modulated such that the interferential superposition of the respective electric fields has the same envelope shape.

In another class of embodiments (claim 19), each plurality of carriers is phase modulated such that the interferential superposition of the respective electric fields is different for each plurality.

In another class of embodiments (claim 20), the excitation envelopes of two different regions of the biological tissue region are independently phase locked with respect to two external synchronization pulse streams.

Another variant exploits the fact that many excitable cells, for example neurons, have varying levels of excitability depending on the direction of the electric field vector in the tissue, e.g. neurons being more excitable when aligned with the field vector. For this purpose, the apparatus comprises
c) a plurality N of at least two electrode pairs, each electrode being formed for application on a surface segment of, or implanted in, said biological body,
d) for each electrode pair of said plurality, a respective electric driving source for generating a respective electric field between electrodes of said pair,
wherein the electrode placements are chosen to obtain essentially orthogonal field vectors in the region of interest and the time-dependent phase difference *φᵢ*(*t*) such that the phase varies between 0 and 360°, providing in two orthogonal directions time dependent excitations in antiphase to each other by virtue of the angular variation in excitability of the tissue. In other directions the variation in the magnitude of the envelope is reduced or negligible. The shape of the envelope in the most favorable directions can be modified by varying the rate of change of phase across the envelope period.

According to another variant, the time-dependent phase difference (φᵢ(t), with i=1 to N) used to obtain the required envelope shape ${φ}_{i} \left(t\right) = \left(i-1\right) ⋅ φ \left(t\right) ⋅ {f}_{m}$ has a frequency fₘ that can be modified, for example using a repetitive input trigger pulse, to synchronize the amplitude modulation envelope phase and frequency with the external input trigger, which could for example be derived from measured neuronal activity. In this variant the rate of reading of the phase data φᵢ(t) is the quantity modified to achieve the carrier envelope phase lock.

Another variant relies on the possibility to independently phase lock the excitation of two different regions of the biological body with respect to two external synchronization pulse streams relating to
c) a plurality (N) of at least two electrode pairs, each electrode being formed for application on a surface segment of, or implanted in, said biological body, with an envelope pulse shape p(t) giving phase modulation data φᵢ(t)
d) and a further plurality (N') of at least two electrode pairs, each electrode being formed for application on a surface segment of, or implanted in, said biological body, operating at a substantially different carrier frequency with an envelope pulse shape p'(t) giving phase modulation data φ'ᵢ(t).

### Brief description of the drawings

The above mentioned and other features and objects of this invention and the manner of achieving them will become more apparent and this invention itself will be better understood by reference to the following description of various embodiments of this invention taken in conjunction with the accompanying drawings, wherein
- Fig. 1: shows a schematic representation of an apparatus according to the invention,
- Fig. 2: shows the superposition of two carrier waves with equal amplitude and orientation,
- Fig. 3: shows the superposition of (a) three, (b) four and (c) five carrier waves with different frequencies where the carrier frequency spacing is equal and all have equal amplitude,
- Fig. 4: shows the relative carrier phase variation in degrees over one envelope period for the case of two carriers with different frequencies producing a sinusoidal amplitude variation,
- Fig. 5: shows the phase shift φ(t) in degrees producing a sinusoidal amplitude variation between consecutively numbered carrier pairs for the cases of (a) three and (b) four carriers,
- Fig. 6: shows the resulting envelope for the superposition of eight equal amplitude carriers (blue dots) using the phase modulation according to the present invention, without envelope correction, versus the desired sinusoidal envelope (solid red),
- Fig. 7: shows phase in degrees as a function of amplitude for the second carrier in a two-carrier scenario, which can be used to translate a given desired envelope amplitude as a function of time p(t) to the required carrier phase difference as a function of time φ(t),

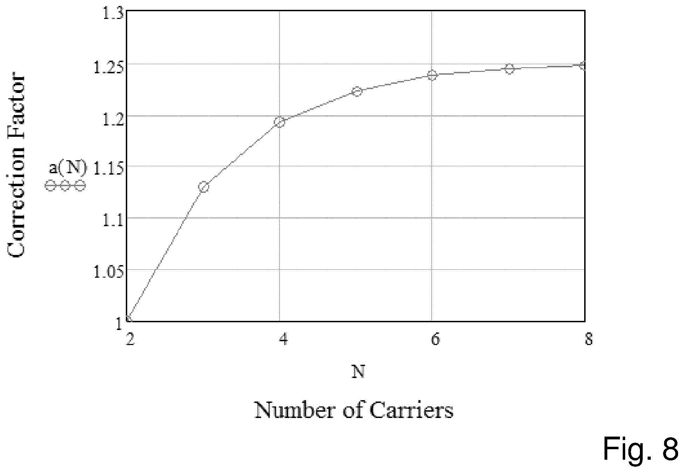

- Fig. 8: shows the compensation factors for the cases N = 2 to N = 8,
- Fig. 9: shows various examples of envelope pulse shape p(t), required phase modulation φ(t) in degrees and the resultant superposition at a location where field vectors are aligned and of equal amplitude,
- Fig. 10: shows a diagram of polarization ellipses illustrating the range of possible polarizations achievable using polarization modulation for the simplest case in 2D with two orthogonal fields E0 and E1 of equal amplitude for a range of different phase shifts, the two linear polarizations as well as circular polarization being highlighted,
- Fig. 11: shows an apparatus that consists of a plurality of sinusoidal sources that can be phase modulated, wherein each source feeds an electric network that converts the output of the source to a differential electric stimulus (voltage or current) of a desired amplitude,
- Fig. 12: shows a DDS architecture for seamless transition from one interferential modulation envelope shape to another,
- Fig. 13: shows an architecture for the Mod Clock of Figures 10 and 11 that will allow phase and frequency locking to an external pulse stream, where the division ratio R is the length of the phase modulation data, and
- Fig. 14: shows an architecture for the readout of the data allowing for direct triggering of the commencement of the readout of the phase modulation data, where the timing of the trigger can impart both frequency and phase to the modulation envelope.

### Detailed description of the invention

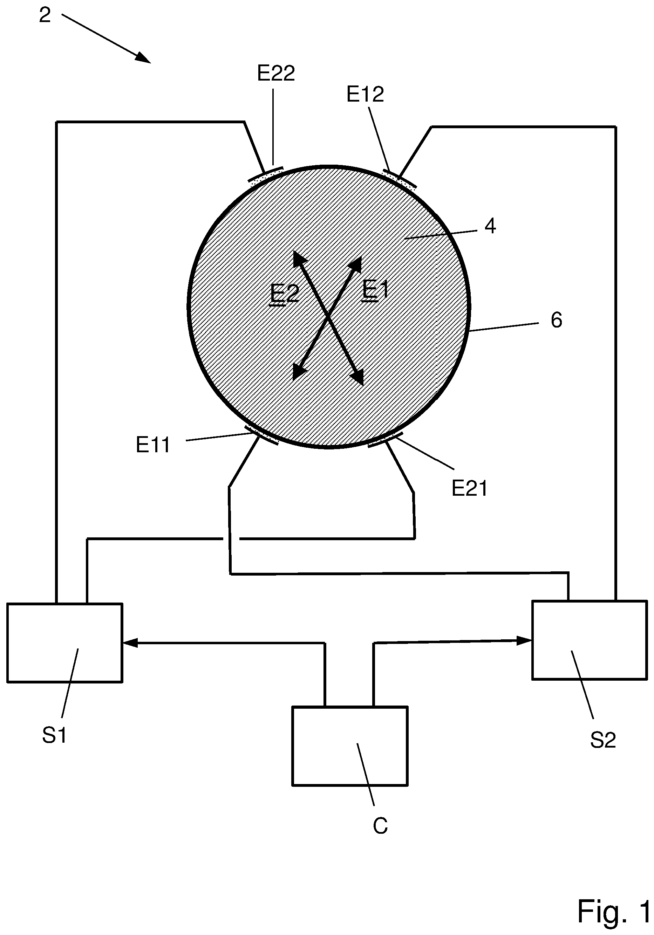

In order to better explain the general principles of the present invention, Fig. 1 shows an overall schematic view of an apparatus 2 for electric field stimulation of biological tissue 4 either in a biological body 6 or in vitro. In the example shown, the apparatus comprises a first electrode pair E11 and E12 and a second electrode pair E21 and E22. Each electrode is applied on a surface segment or implanted in the biological body 6. Each electrode pair is connected to a respective electric driving source S1, S2 for generating a respective electric sinusoidally time dependent field E1 and E2 in the region R between the electrodes of said pair: ${{E}_{¯}}_{i} \left(t, {r}_{¯}\right) = {{E}_{¯}}_{i} \left({r}_{¯}\right) ⋅ sin \left({f}_{0} t+{φ}_{i}\left(t\right)\right)$ (0) with i =1 or 2 and with r being a vector defining position within the overlap region R.

Each one of said time dependent fields has a carrier frequency f₀ and a respective amplitude *Eᵢ*(*r*) and direction.

A resultant time dependent electric field results from vector summation of the respective time dependent fields generated by the electrode pairs *E* (*t, r*) *= E*₁(*t, r*) *+ E*₂(*t, r*). The apparatus further comprises control means C for operating the various electric driving sources S1, S2 in such manner that each one of the respective sinusoidally time dependent electric fields has a time-dependent phase difference (φᵢ(t), with i=1 to N) with respect to a reference phase (φ₀), wherein *φᵢ*(*t*) = (*i* - 1) · *φ*(*t*).

### Background theory

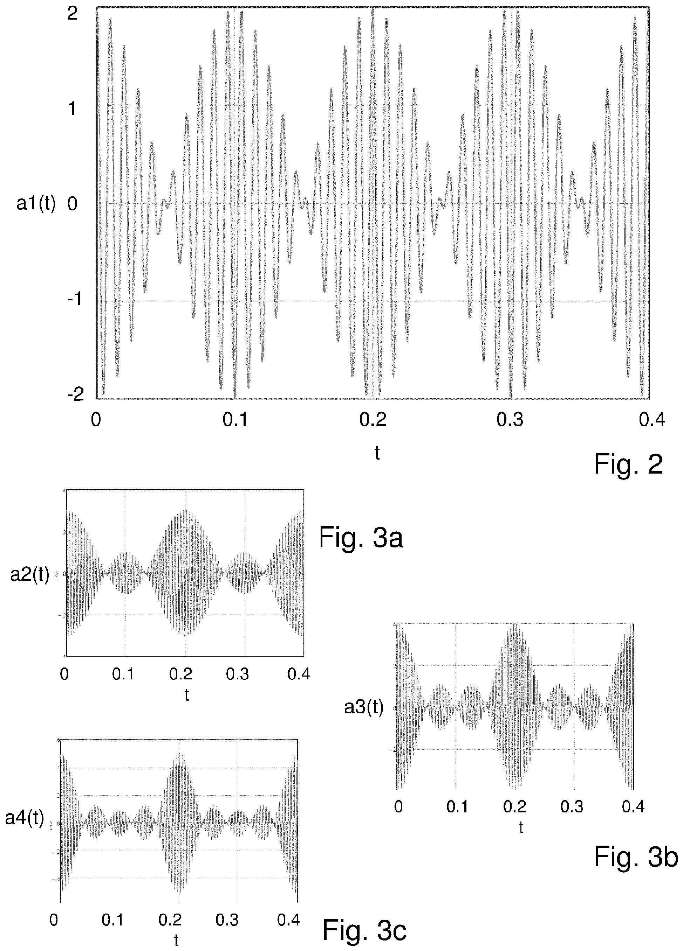

If two carriers of different frequencies are superimposed (summed) the resultant is an amplitude modulated waveform where the amplitude modulation is at Δf the difference in frequency between the two carriers. Consider the case where the carriers have the same amplitude (see Fig. 2) $a 1 \left(t\right) = cos \left({ω}_{0}t\right) + cos \left({ω}_{1}t\right)$ (1)

The envelope is sinusoidal with phase inversion of the resultant at the zero crossings and the amplitude modulation has a modulation index of 1. If the carriers have different amplitudes the modulation index decreases.

In this simplest case, there is no control over the shape of the envelope and the extension to a number of carriers greater than two using the same paradigm by adding carriers at multiples of Δf does not result in similar envelope shapes, but instead adds additional side lobes while narrowing the main signal lobe width (see Fig. 3).

### Phase-Modulation-Approach

Firstly, it is clear that the maximum envelope amplitude always occurs when all carriers are in phase and the field vectors aligned - for the two-carrier case the minimum envelope amplitude occurs when in antiphase (180°). Furthermore from Fig. 3 for more than two carriers it is clear that the maxima still occurs when all are in phase and that other conditions exist for the minima. Considering the cases in Fig. 3, we can extract for each zero magnitude of the modulation envelope the phases of the carriers with respect to the reference carrier (i=1).

**Table 1: Phase shifts in degrees for zero amplitude of the modulation envelope when all carriers have equal amplitude and orientation**

| **Two sinusoids** | **Three sinusoids** | | **Four Sinusoids** | | | **Five sinusoids** | | | |
|---|---|---|---|---|---|---|---|---|---|
| 180 | 120 | -120 | 180 | 90 | -90 | 72 | 144 | -144 | -72 |
| | -120 | 120 | 0 | -180 | 180 | 144 | -72 | 72 | -144 |
| | | | 180 | -90 | 90 | -72 | -144 | 144 | 72 |
| | | | | | | -144 | 72 | -72 | 144 |

We can generalize the phase shift between carriers to achieve cancellation to be 360/N° where N is the number of carriers.

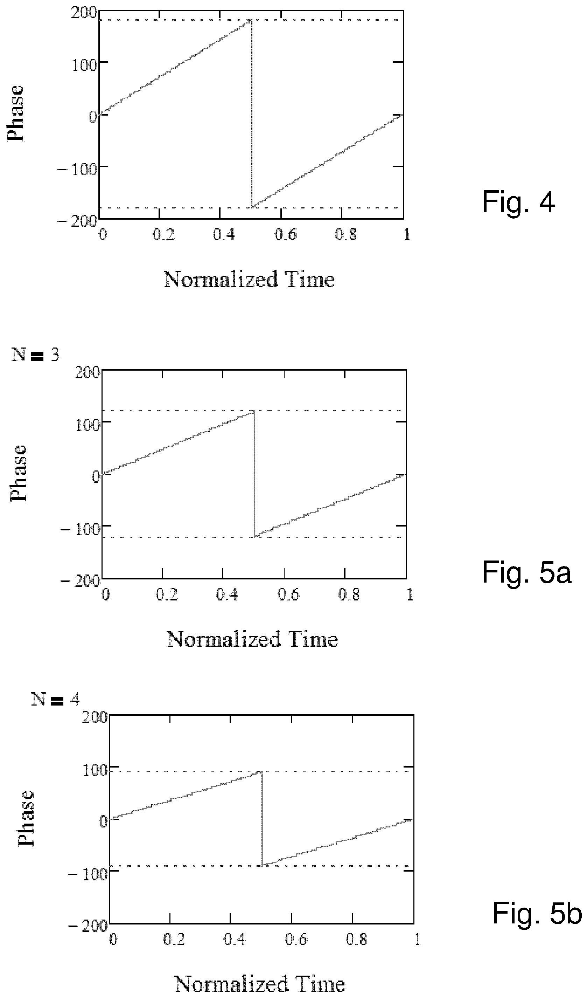

Taking the simplest case of two carriers with different frequencies the resultant envelope is sinusoidal in shape (when the phase inversion is taken into account), with constant frequency difference the relative phase varies linearly over time, see Fig. 4, however, the envelope "pulse" amplitude is not a linear function, see Fig. 2.

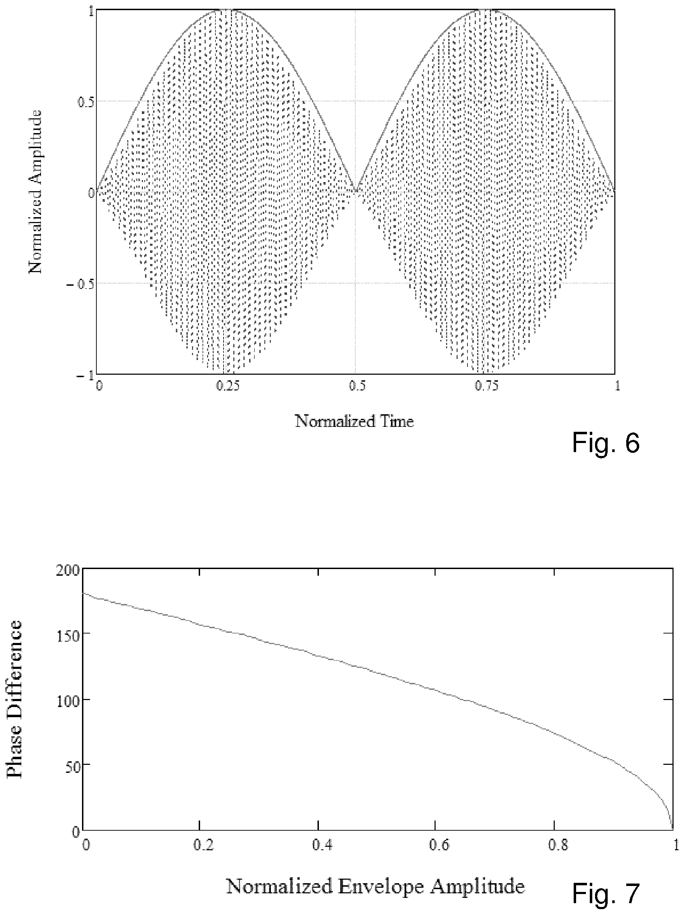

The range of phase variation has to be modified when additional carriers are added to reflect the findings in Table 1, see Fig. 5. All carriers are at the same frequency, the first has no phase modulation, the second has the phase modulation shown in Figure 4 or 5 for the respective number of carriers, then additional carriers have increasing multiples of the phase shift shown in the respective figure. This approach with linear phase difference variation over time results in envelopes approximately the same as that for two carriers of different frequency, note that the required phase shift range decreases inversely with the number of carriers. However, as carriers are added for N>2 the envelope shape departs from the ideal expected if a linear phase increment with time is maintained, see Fig. 6. Hence, a correction factor will need to be applied to achieve exact envelope shapes for the cases where N>2.

Where another envelope shape than that shown in Fig. 2 is required, the mapping of amplitude to phase for amplitudes normalized between 0 and 1 for two carriers is simply the arc cosine of the amplitude as shown in Fig. 7. For amplitudes between 0 and -1 (the resultant carrier inverted) the phase would be negative.

### Method

Here we describe a method of phase modulation allowing the generation of arbitrary envelope pulse shapes from the superposition of an arbitrary number of carrier signals (N ≥ 2) where all the carriers are at the same frequency. The fidelity of the target pulse shape approximation increases with increasing N.

Consider a required pulse p(t) where the amplitude is normalized 0 ≤ p(t) ≤ 1 and the pulse has a duration of 1.

The required phase modulation φ(t) required to give an envelope shape p(t) for N carriers is given by $φ \left(t\right) = \frac{4}{N} ⋅ acos \left(p{\left(t\right)}^{\frac{1}{a \left(N\right)}}\right) ⋅ sign \left(\frac{d}{dt}acos \left(p{\left(t\right)}^{\frac{1}{a \left(N\right)}}\right)\right)$ (2) where φ(t) ranges from -2π/N to +2π/N and the constant a(N) is a correction factor to compensate for the effect seen in Fig. 6, where $a \left(N\right) = 1 + 0.25 \left(1-{e}^{- \frac{N - 2}{1.356}}\right)$ (3)

The values of a(N) can be seen for N in the range 2 to 8 in Fig. 7.

This correction factor does not have to be applied if the exact shape of the envelope is not critical.

In equation (2), the sign of the differential is included to compensate for the fact that the arc cosine function only results in angles between 0 and π radians and not the required -π to +π radians.

The resultant assuming aligned field vectors and equal amplitudes of the carriers can be calculated using equation (4), where all phase shifts are multiples of the calculated phase or, alternatively, as per equation (5), where phase shifts have both positive and negative multipliers and are equally spaced around zero shift. Both result in the same envelope. $w \left(N, t\right) = {\sum }_{i = 0}^{N - 1} cos \left(ωt+i⋅φ\left({f}_{m}⋅t\right)\right)$ (4) $w \left(N, t\right) = {\sum }_{i = 0}^{N - 1} cos \left(ωt+\left(i-\frac{N}{2}\right)⋅φ\left({f}_{m}⋅t\right)\right)$ (5)

### Examples

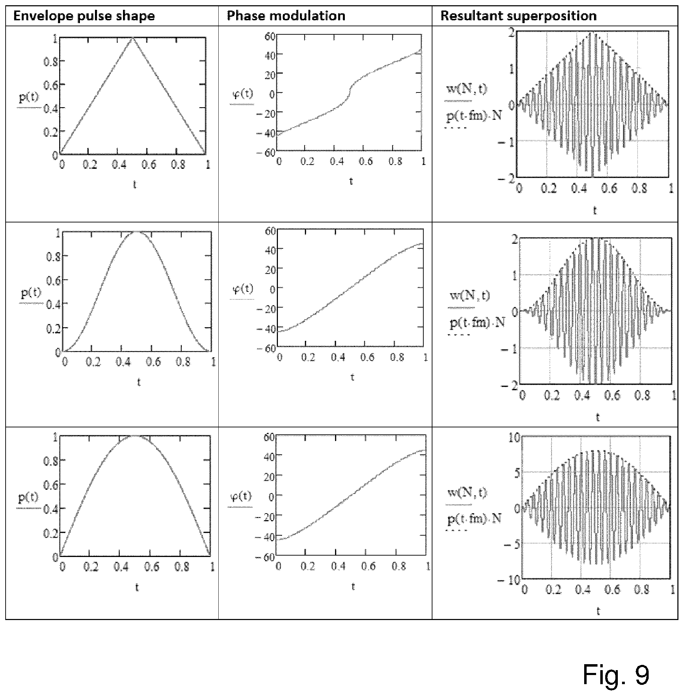

On the left side of Fig. 9, three different examples of normalized envelope amplitude modulation pulse shapes p(t) as a function of normalized time *t* ∈ [0,1) are shown, namely a symmetric triangular pulse (upper trace), a Gaussian-type pulse (middle trace) and a sinusoidal type pulse (bottom trace). Also shown in Fig. 9, in the middle column, are the corresponding phase modulation functions φᵢ(t) and, on the right-hand side, the resultant superpositions for the case of N = 8 superposed phase modulated electric fields, which form the interferential modulation of the fast-oscillating signals with an envelope substantially matching the desired normalized envelope pulse shape.

### Polarization modulation method

Here we describe a non-claimed method of electric field stimulation of biological tissues that exhibit variation in excitability with excitation field angle by means of phase modulation and electrode placement to produce in the target tissue electric fields of time dependent polarization. The placement of each electrode pair is chosen such that they generate respective electric field between the electrodes with a given direction in the target region. The time dependent phase difference between each pair of electrodes in the plurality is chosen such that the superposition of the fields results in a time dependent variation in polarization.

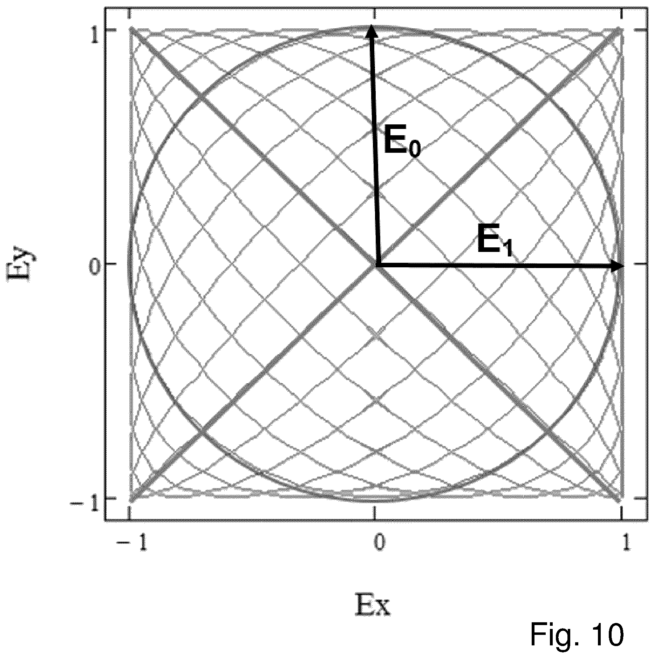

In the simplest form two electrode pairs are placed such that their respective electric fields in the target region are orthogonal to each other. Then, as the phase difference progresses from 0 to 360°, the electric field polarization will vary from linear through elliptical to circular to elliptical and back to linear, whereby the varying alignment of fields within tissues will result in varying levels of excitation depending on the direction of the electric field vector, see Fig. 10. Where the phase difference is time dependent, the tissue will experience a time dependent excitation of the tissue.

The time-dependent phase differences *φᵢ*(*t*) can be chosen such that the rate of change of electric field polarization is modified such that the product of the angular excitability and polarization angle result in a desired excitation as a function of time:$Excitation \left(t\right) = Excitability \left(Θ, ϕ\right) \cdot E \left(Θ, ϕ, t\right)$ where Θ and Φ are the spherical coordinates for vectors

### Apparatus

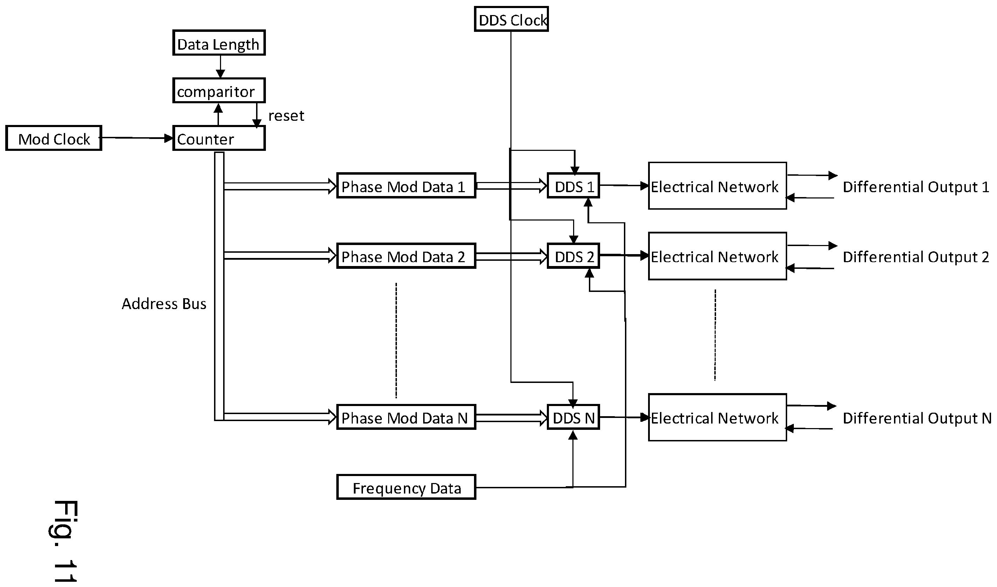

The exemplary apparatus shown in Fig. 11 comprises a plurality of N sinusoidal sources configured as outputs of respective direct digital synthesizers (DDS) that can be phase modulated. All of these source channels run from common digital clock and can be synchronized to have the same initial frequency and phase. A calculated phase modulation can then be applied synchronously to all channels, each block of phase modulation data has the same data length and the appropriate values φᵢ(t), where i=1 to N. Time t is handled as discrete variable with steps in increments of 1/D, where D is the data length, and the data is for one normalized period of 1. The required modulation envelope period tₘ is defined by the modulation clock f_{data} which is set to D/tₘ and can be varied at any time to change the envelope frequency while maintaining continuity of both carrier and envelope.

Each phase modulated sinusoidal source feeds an electrical network that converts the output of the source to a differential electrical stimulus (voltage or current) of a desired amplitude that can then be used to excite a pair of electrodes (implanted or in surface contact with the biological body) in order to induce electric fields between the electrodes inside the biological body.

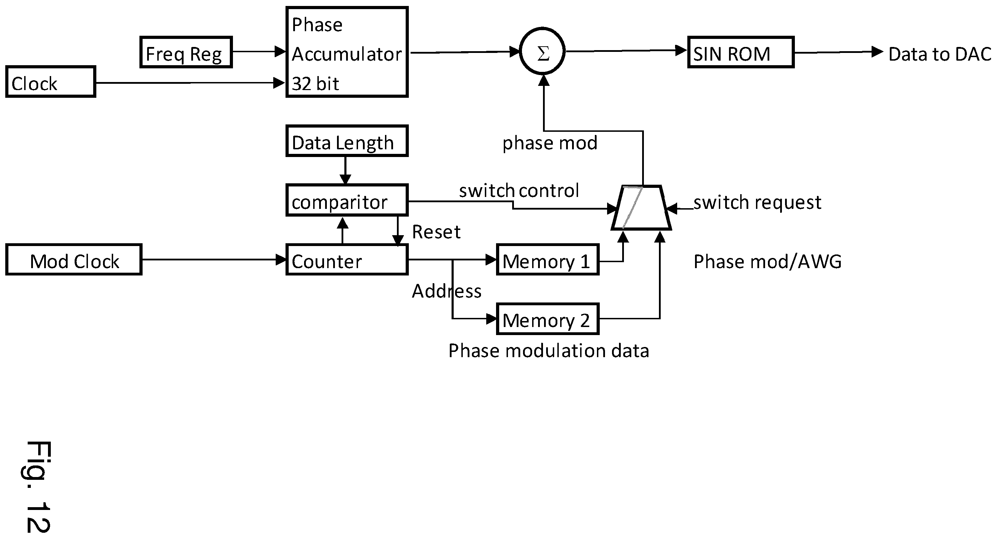

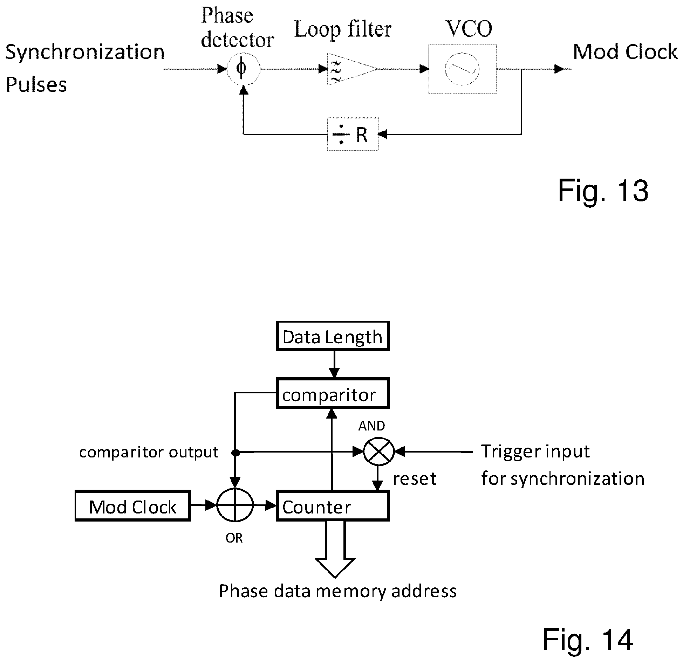

Another refinement, as illustrated in Fig. 12, can introduce a second block of phase modulation data and switched between the two as user requires, providing switching over takes place only when the address counter is resetting to zero, i.e. at the end of a full cycle of data, this will ensure that the transition is controlled. The respective blocks of data can be written at any time providing that block of data is not in active use. f_{data} can be modified in light of an external input trigger pulse train to synchronize the amplitude modulation envelope phase and frequency with, for example, measured neuronal activity. This can be achieved by means of a phase locked loop which can be implemented in either hardware or digital domains, as shown in Fig. 13, where the divider R is equal to the data length or by direct triggering of the commencement of the readout of the phase modulation data, as shown in Fig. 14. The readout is paused at the end of the data until the next trigger, and the repetition rate is limited such that it ensures the data has all been read out prior to the next trigger event.

## Claims

1. An apparatus (2) for electric field stimulation of a biological tissue region (4), the apparatus comprising:
a) a plurality (N) of at least two electrode pairs (E11, E12; E21, E22), each electrode being suited for application on a surface segment of, or implantation into, said biological tissue region;
b) for each electrode pair of said plurality, a respective electric driving source (S1, S2) for generating a respective electric field between electrodes of said pair,
wherein each electric driving source is configured to generate a respective sinusoidally time dependent electric field (E1, E2) having a respective carrier frequency and a respective amplitude,
whereby a resultant time dependent electric field having a phase modulation frequency is formed in a region wherein said respective electric fields spatially overlap,
**characterized in that**
the apparatus comprises control means (C) for operating all of said electric driving sources in such manner that
- all of said respective frequencies are identical to one preselected carrier frequency (f₀), the carrier frequency (f₀) being selected from 500 Hz to 1 MHz,
- each one of said respective sinusoidally time dependent electric fields has a defined normalized time-dependent phase difference for one cycle (φᵢ(t), with i=1 to N) with respect to a reference phase (φ₀ = 0), wherein ${φ}_{i} \left(t\right) = \left(i-1\right) ⋅ φ \left(t\right)$
- where *φ*(*t*) varies from -2π/N to +2π/N
- and, if the phase modulation frequency is desired to be fₘ, then the time-dependent phase difference is given by ${φ}_{i} \left(t\right) = \left(i-1\right) ⋅ φ \left(t\right) ⋅ {f}_{m}$

2. The apparatus according to claim 1, wherein said plurality (N) of electrode pairs is at least three.

3. The apparatus according to claim 1 or 2, wherein said control means comprise:
i) means for inputting or selecting a pulse shape function p(t) in a normalized time range t = 0 to 1,
ii) means for determining said phase modulation function φ(t) in said normalized time range,
iii) means for generating said respective sinusoidally time dependent electric fields, each having a preselected time-dependent phase difference (φᵢ(t), with i=1 to N) with respect to a reference phase (φ₀), wherein: ${φ}_{i} \left(t\right) = \left(i-1\right) ⋅ φ \left(t\right) ⋅ {f}_{m}$
whereby the resultant electric field in a region wherein the respective aligned electric field vectors spatially overlap has a temporal envelope shape corresponding to said normalized envelope pulse shape function p(t) in said normalized time range.

4. The apparatus according to claim 3, wherein the phase modulation function φ(t) is: $φ \left(t\right) = \frac{4}{N} ⋅ acos \left(p{\left(t\right)}^{\frac{1}{a \left(N\right)}}\right) ⋅ sign \left(\frac{d}{dt}acos \left(p{\left(t\right)}^{\frac{1}{a \left(N\right)}}\right)\right)$ wherein a(N) is an envelope correction factor.

5. The apparatus according to claim 3, wherein the phase modulation function φ(t) is: $φ \left(t\right) = \pm abs \left[\frac{4}{N}⋅acos \left(p{\left(t\right)}^{\frac{1}{a \left(N\right)}}\right)⋅sign\left(\frac{d}{dt}acos \left(p{\left(t\right)}^{\frac{1}{a \left(N\right)}}\right)\right)\right]$ wherein a(N) is an envelope correction factor.

6. The apparatus according to claim 4 or 5, wherein the envelope correction factor a(N) is $a \left(N\right) = 1 + 0.25 \left(1-{e}^{- \frac{N - 2}{1.356}}\right)$

7. The apparatus according to claim 4 or 5, wherein a(N) = 1.

8. The apparatus according to one of claims 3 to 7, wherein said field generating means iii) are configured to carry out a temporally repetitive sequence of stimulation cycles.

9. The apparatus according to claim 8, with first means for inputting or selecting a first normalized envelope pulse shape function p1(t) in a first normalized time range and second means for inputting or selecting a second normalized envelope pulse shape function p2(t) in a second normalized time range that switches to follow directly after the first normalized time range upon receiving a command to change envelope shape, wherein said first and second means are configured to smoothly switch from said first normalized envelope pulse shape function p1(t) to said second normalized envelope pulse shape function p2(t) synchronously for all electrode pairs of a plurality.

10. The apparatus according to claim 9, configured in such manner that phase modulation data is read out of a phase modulation memory block at a controlled rate so as to ensure both carrier and envelope phase continuity when modifying the phase modulation frequency (fₘ).

11. The apparatus according to one of claims 1 to 10, configured in such manner that the phase modulation frequency (fₘ) can be modified to be synchronized with an external input trigger pulse train.

12. The apparatus according to claim 1 or 2, configured for generating two different sham exposure states in a biological tissue of interest, in which no time varying interferential envelope modulation p(t) is present, wherein in the first sham exposure state the carrier frequency induced fields in the biological tissue minimize the total field magnitude and wherein in the second sham exposure state the carrier frequency induced fields in the biological tissue all are in phase.

13. The apparatus according to claim 1 or 2, configured in such manner that the combination of
a) the placement of each electrode pair of said plurality is chosen such that they generate a respective electric field between each pair of said electrodes with a given direction in the target region
b) the time dependent phase difference between each pair of electrodes in the plurality is chosen such that the superposition of the fields results in a time dependent variation in polarization of from linear through elliptical and back to linear whereby appropriate alignment of fields within tissues having varying levels of excitability depending on the direction of the electric field vector experience a time dependent excitation of the tissue.

14. The apparatus according to claim 1 or 2, configured in such manner that the time-dependent phase differences *φᵢ*(*t*) is such that the rate of change of electric field polarization is modified such that the product of the angular excitability and polarization angle result in a desired excitation as a function of time.

15. The apparatus according to one of claims 1 to 14, wherein said electric driving sources each comprise a digital synthesizer, all of said digital synthesizers running from a common clock so as to be synchronizable to said common reference phase (φ₀).

16. The apparatus according to claim 15, wherein each one of said electric driving sources comprises an electric network that converts an output signal of the respective digital synthesizer to a corresponding differential electric voltage stimulus or current stimulus of a desired amplitude that excites an associated electrode pair.

17. The apparatus according to one of claims 1 to 16, comprising
c) a further plurality (N') of at least two electrode pairs, each electrode being formed for application on a surface segment of, or implantation into, said biological tissue region,
d) for each electrode pair of said further plurality, a respective further electric driving source for generating a respective further electric field between electrodes of said pair,
wherein each further electric driving source is configured to generate a respective sinusoidally time dependent further electric field having a respective further carrier frequency and a respective further amplitude,
whereby a resultant time dependent further electric field is formed in a region wherein said further respective electric fields spatially overlap,
the apparatus comprising further control means for operating all of said further electric driving sources in such manner that
- all of said respective further carrier frequencies are identical to one preselected further carrier frequency (f₀'), the further carrier frequency (f₀') being selected from 500 Hz to 1 MHz,
- each one of said respective further sinusoidally time dependent electric fields has a defined normalized time-dependent further phase difference for one cycle (φᵢ'(t), with i=1 to N') with respect to a further reference phase (φ₀' = 0), wherein ${φ}_{i} ′ \left(t\right) = \left(i-1\right) ⋅ φ ′ \left(t\right)$
- and, if the frequency of the phase modulation is desired to be fₘ, then the time-dependent further phase difference is given by ${φ}_{i} ′ \left(t\right) = \left(i-1\right) ⋅ φ ′ \left(t\right) ⋅ f {′}_{m}$
wherein said carrier frequency (f₀) and said further carrier frequency (f₀') are spaced apart by a frequency difference (Δf) which is at least 500 Hz.

18. The apparatus according to claim 17, configured in such manner that the carrier frequency (f₀) and the further carrier frequency (f₀') are phase modulated such that the interferential superposition of their respective electric fields has the same envelope shape and frequency.

19. The apparatus according to claim 17, configured in such manner that the carrier frequency (f₀) and the further carrier frequency (f₀') are phase modulated such that the interferential superposition of their respective electric fields is different.

20. The apparatus according to claim 19, configured in such manner that the excitation envelopes of two different regions of the biological tissue region are independently phase locked with respect to two external synchronization pulse streams.

## Patentansprüche

1. Vorrichtung (2) zur elektrischen Feldstimulation eines biologischen Gewebebereichs (4), wobei die Vorrichtung umfasst:
a) eine Vielzahl (N) von mindestens zwei Elektrodenpaaren (E11, E12; E21, E22), wobei jede Elektrode zur Anwendung auf einem Oberflächensegment des biologischen Gewebebereichs oder zur Implantation in diesen besagten Gewebebereich geeignet ist;
b) für jedes Elektrodenpaar der besagten Vielzahl, eine jeweilige elektrische Ansteuerungsquelle (S1, S2) zum Erzeugen eines jeweiligen elektrischen Feldes zwischen den Elektroden des besagten Paares vorgesehen,
wobei jede elektrische Ansteuerungsquelle so ausgelegt ist, dass sie ein jeweiliges sinusförmig zeitabhängiges elektrisches Feld (E1, E2) mit einer jeweiligen Trägerfrequenz und einer jeweiligen Amplitude erzeugt,
wodurch in einem Bereich, in dem sich die jeweiligen elektrischen Felder räumlich überlappen, ein resultierendes zeitabhängiges elektrisches Feld mit einer Phasenmodulationsfrequenz erzeugt wird,
**dadurch gekennzeichnet, dass**
die Vorrichtung Steuermittel (C) zum Betreiben aller genannten elektrischen Antriebsquellen derart umfasst, dass
- alle besagten jeweiligen Frequenzen mit einer vorgewählten Trägerfrequenz (f₀) identisch sind, wobei die Trägerfrequenz (f₀) im Bereich von 500 Hz bis 1 MHz gewählt wird,
- jedes der besagten sinusförmig zeitabhängigen elektrischen Felder über einen Zyklus eine definierte normierte zeitabhängige Phasendifferenz (φᵢ(t), mit i=1 bis N) bezüglich einer Referenzphase (φ₀ = 0) aufweist, wobei ${φ}_{i} \left(t\right) = \left(i-1\right) ⋅ φ \left(t\right)$
- wobei *φ*(*t*) von -2π/N bis +2π/N variiert
- und, falls die Phasenmodulationsfrequenz fₘ betragen soll, die zeitabhängige Phasendifferenz gegeben ist durch: ${φ}_{i} \left(t\right) = \left(i-1\right) ⋅ φ \left(t\right) ⋅ {f}_{m}$

2. Vorrichtung nach Anspruch 1, wobei die besagte Vielzahl (N) der Elektrodenpaare mindestens drei beträgt.

3. Vorrichtung nach Anspruch 1 oder 2, wobei die besagten Steuermittel umfassen:
i) Mittel zum Eingeben oder Auswählen einer Impulsformfunktion p(t) in einem normierten Zeitbereich t = 0 bis 1,
ii) Mittel zur Bestimmung der besagten Phasenmodulationsfunktion φ(t) im besagten normierten Zeitbereich,
iii) Mittel zum Erzeugen der besagten jeweiligen sinusförmigen, zeitabhängigen elektrischen Felder, die jeweils eine voreingestellte zeitabhängige Phasendifferenz (φᵢ(t) mit i=1 bis N) bezüglich einer Referenzphase (φ₀) aufweisen, wobei: ${φ}_{i} \left(t\right) = \left(i-1\right) ⋅ φ \left(t\right) ⋅ {f}_{m}$
wodurch das resultierende elektrische Feld in einem Bereich, in dem sich die jeweiligen ausgerichteten elektrischen Feldvektoren räumlich überlappen, eine zeitliche Hüllkurvenform aufweist, die der besagten normierten Hüllenkurven-Impulsformfunktion p(t) im besagten normierten Zeitbereich entspricht.

4. Vorrichtung nach Anspruch 3, wobei die Phasenmodulationsfunktion φ(t) gegeben ist durch: $φ \left(t\right) = \frac{4}{N} ⋅ acos \left(p{\left(t\right)}^{\frac{1}{a \left(N\right)}}\right) ⋅ sign \left(\frac{d}{dt}acos \left(p{\left(t\right)}^{\frac{1}{a \left(N\right)}}\right)\right)$ wobei a(N) ein Hüllkurvenkorrekturfaktor ist.

5. Vorrichtung nach Anspruch 3, wobei die Phasenmodulationsfunktion φ(t) gegeben ist durch: $φ \left(t\right) = \pm abs \left[\frac{4}{N}⋅acos \left(p{\left(t\right)}^{\frac{1}{a \left(N\right)}}\right)⋅sign\left(\frac{d}{dt}acos \left(p{\left(t\right)}^{\frac{1}{a \left(N\right)}}\right)\right)\right]$ wobei a(N) ein Hüllkurvenkorrekturfaktor ist.

6. Vorrichtung nach Anspruch 4 oder 5, wobei der Hüllkurvenkorrekturfaktor a(N) gegeben ist durch $a \left(N\right) = 1 + 0.25 \left(1-{e}^{- \frac{N - 2}{1.356}}\right)$

7. Vorrichtung nach Anspruch 4 oder 5, wobei a(N) = 1 ist.

8. Vorrichtung nach einem der Ansprüche 3 bis 7, wobei die besagten Felderzeugungsmittel iii) so ausgelegt sind, dass sie eine sich zeitlich wiederholende Abfolge von Stimulationszyklen ausführen.

9. Vorrichtung nach Anspruch 8, umfassend erste Mittel zum Eingeben oder Auswählen einer ersten normierten Hüllkurvenimpulsformfunktion p1(t) in einem ersten normierten Zeitbereich und zweite Mittel zum Eingeben oder Auswählen einer zweiten normierten Hüllkurvenimpulsformfunktion p2(t) in einem zweiten normierten Zeitbereich, die unmittelbar nach dem ersten normierten Zeitbereich einsetzt, sobald ein Befehl zur Änderung der Hüllkurvenform empfangen wird, wobei die besagten ersten und zweiten Mittel so ausgelegt sind, dass sie für alle Elektrodenpaare einer Vielzahl synchron und stufenlos von der ersten normierten Hüllkurven-Impulsformfunktion p1(t) zur zweiten normierten Hüllkurven-Impulsformfunktion p2(t) übergehen.

10. Vorrichtung nach Anspruch 9, die derart ausgelegt ist, dass Phasenmodulationsdaten mit kontrollierter Geschwindigkeit aus einem Phasenmodulationsspeicherblock ausgelesen werden, um die Kontinuität sowohl der Träger- als auch der Hüllkurvenphase bei einer Änderung der Phasenmodulationsfrequenz (fₘ) zu gewährleisten.

11. Vorrichtung nach einem der Ansprüche 1 bis 10, die derart ausgelegt ist, dass die Phasenmodulationsfrequenz (fₘ) so verändert werden kann, dass sie mit einer externen Eingangs-Triggerimpulsfolge synchronisiert ist.

12. Vorrichtung nach Anspruch 1 oder 2, die ausgelegt ist zur Erzeugung zweier verschiedener Scheinexpositionszustände in einem biologischen Gewebe von Interesse, in denen keine zeitlich veränderliche Interferenzhüllkurvenmodulation p(t) vorliegt, wobei im ersten Scheinexpositionszustand die durch die Trägerfrequenz induzierten Felder im biologischen Gewebe die Gesamtfeldstärke minimieren und wobei im zweiten Scheinexpositionszustand die durch die Trägerfrequenz induzierten Felder im biologischen Gewebe alle in Phase sind.

13. Vorrichtung nach Anspruch 1 oder 2, die derart ausgelegt ist, dass die Kombination aus
a) die Anordnung eines jeden Elektrodenpaares der besagten Vielzahl so gewählt ist, dass zwischen jedem Elektrodenpaar ein entsprechendes elektrisches Feld mit einer vorgegebenen Richtung im Targetbereich erzeugt wird;
b) die zeitabhängige Phasendifferenz zwischen jedem Elektrodenpaar der Vielzahl so gewählt ist, dass die Überlagerung der Felder zu einer zeitabhängigen Änderung der Polarisation von linear über elliptisch und zurück zu linear führt,
wodurch eine geeignete Ausrichtung der Felder in Geweben mit unterschiedlicher Erregbarkeit in Abhängigkeit von der Richtung des elektrischen Feldvektors eine zeitabhängige Anregung des Gewebes bewirkt.

14. Vorrichtung nach Anspruch 1 oder 2, die derart ausgelegt ist, dass die zeitabhängigen Phasendifferenzen *φᵢ*(*t*) so beschaffen sind, dass die Änderungsrate der elektrischen Feldpolarisation so verändert wird, dass das Produkt aus Winkelanregbarkeit und Polarisationswinkel zu einer gewünschten Anregung als Funktion der Zeit führt.

15. Vorrichtung nach einem der Ansprüche 1 bis 14, wobei die besagten elektrischen Ansteuerungsquellen jeweils einen digitalen Synthesizer umfassen, wobei alle besagten digitalen Synthesizer von einem gemeinsamen Taktgeber angesteuert werden, so dass sie mit der gemeinsamen Referenzphase (φ₀) synchronisierbar sind.

16. Vorrichtung nach Anspruch 15, wobei jede der besagten elektrischen Ansteuerungsquellen ein elektrisches Netzwerk umfasst, das ein Ausgangssignal des jeweiligen digitalen Synthesizers in einen entsprechenden differentiellen elektrischen Spannungs- oder Stromimpuls mit einer gewünschten Amplitude umwandelt, der ein zugehöriges Elektrodenpaar anregt.

17. Vorrichtung nach einem der Ansprüche 1 bis 16, umfassend
c) eine weitere Vielzahl (N') von mindestens zwei Elektrodenpaaren, wobei jede Elektrode zur Anwendung auf einem Oberflächensegment oder zur Implantation in den besagten biologischen Gewebebereich ausgebildet ist;
d) für jedes Elektrodenpaar der besagten weiteren Vielzahl eine jeweilige weitere elektrische Ansteuerungsquelle zur Erzeugung eines jeweiligen weiteren elektrischen Feldes zwischen den Elektroden des besagten Paares,
wobei jede weitere elektrische Ansteuerungsquelle so ausgelegt ist, dass sie ein jeweiliges sinusförmig zeitabhängiges weiteres elektrisches Feld mit einer jeweiligen weiteren Trägerfrequenz und einer jeweiligen weitere Amplitude erzeugt, wodurch in einem Bereich, in dem sich die jeweiligen weiteren elektrischen Felder räumlich überlappen, ein resultierendes zeitabhängiges weiteres elektrisches Feld erzeugt wird,
wobei die Vorrichtung Steuermittel zum Betreiben aller genannten weiteren elektrischen Antriebsquellen derart umfasst, dass
- alle besagten jeweiligen weiteren Trägerfrequenzen mit einer vorgewählten Trägerfrequenz (f₀') identisch sind, wobei die weitere Trägerfrequenz (f₀') im Bereich von 500 Hz bis 1 MHz gewählt wird,
- jedes der besagten weiteren sinusförmig zeitabhängigen elektrischen Felder für einen Zyklus eine definierte normierte zeitabhängige weitere Phasendifferenz (φᵢ'(t), mit i=1 bis N') bezüglich einer weiteren Referenzphase (φ₀' = 0) aufweist, wobei ${φ}_{i} ′ \left(t\right) = \left(i-1\right) ⋅ φ ′ \left(t\right)$
- und, falls die Phasenmodulationsfrequenz fₘ betragen sollt, die zeitabhängige weitere Phasendifferenz gegeben ist durch: ${φ}_{i} ′ \left(t\right) = \left(i-1\right) ⋅ φ ′ \left(t\right) ⋅ f {′}_{m}$
wobei die besagte Trägerfrequenz (f₀) und die besagte weitere Trägerfrequenz (f₀') durch eine Frequenzdifferenz (Δf) voneinander beabstandet sind, die mindestens 500 Hz beträgt.

18. Vorrichtung nach Anspruch 17, welche derart ausgelegt ist, dass die Trägerfrequenz (f₀) und die weitere Trägerfrequenz (f₀') so phasenmoduliert sind, dass die Interferenzüberlagerung ihrer jeweiligen elektrischen Felder dieselbe Hüllkurvenform und Frequenz aufweist.

19. Vorrichtung nach Anspruch 17, welche derart ausgelegt ist, dass die Trägerfrequenz (f₀) und die weitere Trägerfrequenz (f₀') so phasenmoduliert sind, dass die Interferenzüberlagerung ihrer jeweiligen elektrischen Felder unterschiedlich ist.

20. Vorrichtung nach Anspruch 19, welche derart ausgelegt ist, dass die Anregungshüllkurven zweier verschiedener Bereiche des biologischen Gewebes unabhängig voneinander in Bezug auf zwei externe Synchronisationsimpulse phasengekoppelt sind.

## Revendications

1. Un appareil (2) pour la stimulation par champ électrique d'une région de tissu biologique (4), comprenant :
a) une pluralité (N) d'au moins deux paires d'électrodes (E11, E12; E21, E22), chaque électrode étant adaptée à une application sur un segment de surface de ladite région de tissu biologique, ou à une implantation dans ladite région de tissu biologique;
b) pour chaque paire d'électrodes de ladite pluralité, une source d'excitation électrique respective (S1, S2) pour générer un champ électrique respectif entre les électrodes de ladite paire.
dans lequel chaque source d'excitation électrique est configurée pour générer un champ électrique sinusoïdal dépendant du temps (E1, E2), ayant une fréquence porteuse respective et une amplitude respective,
un champ électrique résultant dépendant du temps et présentant une fréquence de modulation de phase ainsi étant formé dans une région où lesdits champs électriques respectifs se superposent spatialement,
**caractérisé en ce que**
l'appareil comprend des moyens de commande (C) permettant de faire fonctionner toutes desdites sources d'excitation électrique de telle sorte que:
- toutes lesdites fréquences respectives sont identiques à une fréquence porteuse présélectionnée (f0), la fréquence porteuse étant choisie entre 500 Hz et 1 MHz,
- chacun desdits champs électriques sinusoïdaux dépendant du temps respectifs présente une différence de phase normalisée dépendant du temps définie, pour un cycle (φᵢ(t), avec i=1 to N) par rapport à une phase de référence (φ₀ = 0), avec ${φ}_{i} \left(t\right) = \left(i-1\right) ⋅ φ \left(t\right)$
- où *φ*(*t*) varie de -2π/N à +2π/N
- et, si la fréquence de modulation de phase souhaitée est fₘ, alors la différence de phase dépendant du temps est donnée par ${φ}_{i} \left(t\right) = \left(i-1\right) ⋅ φ \left(t\right) ⋅ {f}_{m}$

2. L'appareil selon la revendication 1, dans lequel ladite pluralité (N) de paires d'électrodes est d'au moins trois.

3. L'appareil selon la revendication 1 ou 2, dans lequel lesdits moyens de commande comprennent:
i) des moyens pour entrer ou sélectionner une fonction de forme d'impulsion p(t) dans un intervalle de temps normalisé t = 0 to 1,
ii) des moyens pour déterminer ladite fonction de modulation de phase φ(t) dans ladite plage de temps normalisée,
iii) des moyens pour générer lesdits champs électriques sinusoïdaux et dépendants du temps respectifs, chacun présentant une différence de phase prédé-finie (φᵢ(t), avec i=1 à N) par rapport à une phase de référence (φ₀), dans lequel: ${φ}_{i} \left(t\right) = \left(i-1\right) ⋅ φ \left(t\right) ⋅ {f}_{m}$
le champ électrique résultant dans une région où les vecteurs de champ électrique respectifs alignés se superposent spatialement présentant une forme d'enveloppe temporelle correspondant à ladite fonction de forme d'impulsion d'enveloppe normalisée p(t) dans ladite plage de temps normalisée.

4. L'appareil selon la revendication 3, dans lequel la fonction de modulation de phase φ(t) est: $φ \left(t\right) = \frac{4}{N} ⋅ acos \left(p{\left(t\right)}^{\frac{1}{a \left(N\right)}}\right) ⋅ sign \left(\frac{d}{dt}acos \left(p{\left(t\right)}^{\frac{1}{a \left(N\right)}}\right)\right)$ dans lequel a(N) est un facteur de correction d'enveloppe.

5. L'appareil selon la revendication 3, dans lequel la fonction de modulation de phase φ(t) est: $φ \left(t\right) = \pm abs \left[\frac{4}{N}⋅acos \left(p{\left(t\right)}^{\frac{1}{a \left(N\right)}}\right)⋅sign\left(\frac{d}{dt}acos \left(p{\left(t\right)}^{\frac{1}{a \left(N\right)}}\right)\right)\right]$ dans lequel a(N) est un facteur de correction d'enveloppe.

6. L'appareil selon la revendication 4 ou 5, dans lequel le facteur de correction d'enveloppe a(N) est $a \left(N\right) = 1 + 0.25 \left(1-{e}^{- \frac{N - 2}{1.356}}\right)$

7. L'appareil selon la revendication 4 or 5, dans lequel a(N) = 1.

8. L'appareil selon l'une des revendications 3 à 7, dans lequel lesdits moyens de génération de champ iii) sont configurés pour exécuter une séquence temporelle répétitive de cycles de stimulation.

9. L'appareil selon la revendication 8, comportant premiers moyens pour entrer ou sélectionner une première fonction de forme d'impulsion d'enveloppe normalisée p1(t) dans une première plage temporelle normalisée et deuxièmes moyens pour entrer ou sélectionner une deuxième fonction de forme d'impulsion d'enveloppe normalisée p2(t) dans une deuxième plage temporelle normalisée qui passe directement après la première plage temporelle normalisée lors de la réception d'une commande de changement de forme d'enveloppe, dans lequel lesdits premiers et deuxièmes moyens sont configurés pour passer en douceur de ladite première fonction de forme d'enveloppe normalisée p1(t) à ladite deuxième fonction de forme d'enveloppe normalisée p2(t) de manière synchrone pour toutes les paires d'électrodes d'une pluralité.

10. L'appareil selon la revendication 9, configuré de telle manière que les données de modulation de phase soient lues à partir d'un bloc de mémoire de modulation de phase à une fréquence contrôlée, afin d'assurer la continuité de la phase de la porteuse et de l'enveloppe lors de la modification de la fréquence de modulation de phase (fₘ).

11. Appareil selon l'une des revendications 1 à 10, configuré de telle manière que la fréquence de modulation de phase (fₘ) puisse être modifiée pour être synchronisée avec un train d'impulsions de déclenchement d'entrée externe.

12. L'appareil selon la revendication 1 ou 2, configuré pour générer deux états d'exposition simulée différents dans lesquels aucune modulation d'enveloppe interférentielle variable dans le temps p(t) n'est présente, dans lequel, dans le premier état d'exposition simulée, les champs induits par la fréquence porteuse dans le tissu biologique minimisent l'amplitude totale du champ et dans lequel, dans le deuxième état d'exposition simulée, les champs induits par la fréquence porteuse dans le tissu biologique sont tous en phase.

13. L'appareil selon la revendication 1 ou 2, configuré de telle manière que la combinaison de
a) le placement de chaque paire d'électrodes de ladite pluralité est choisi de telle manière qu'un champ électrique respectif entre chaque paire desdites électrodes avec une direction donnée dans la région cible soit généré
b) la différence de phase dépendante entre chaque paire d'électrodes de la pluralité est choisie de telle manière que la superposition des champs entraîne une variation de la polarisation en fonction du temps de linéaire à elliptique et de retour à linéaire,
ainsi qu'un alignement approprié des champs dans les tissus ayant des niveaux d'excitabilité variables en fonction de la direction du vecteur de champ électrique provoque une excitation dépendante du temps du tissu.

14. L'appareil selon la revendication 1 ou 2, configuré de telle manière que les différences de phase en fonction du temps *φᵢ*(*t*) soient de telle manière que la fréquence de changement de la polarisation du champ électrique soit modifiée de telle manière que produit de l'excitabilité angulaire et de l'angle de polarisation produise l'excitation souhaitée en fonction du temps.

15. Appareil selon l'une des revendications 1 à 14, dans lequel lesdites sources d'excitation électriques comprennent chacune un synthétiseur numérique, tous lesdits synthétiseurs numériques fonctionnant à partir d'une horloge commune afin d'être synchronisés sur ladite phase de référence commune (φ₀).

16. L'appareil selon la revendication 15, dans lequel chacune desdites sources d'excitation électriques comprend un réseau électrique qui convertit un signal de sortie du synthétiseur numérique correspondant en un stimulus de tension électrique différentielle ou en un stimulus de courant correspondant d'une amplitude souhaitée, qui excite une paire d'électrodes associée.

17. L'appareil selon l'une des revendications 1 à 16, comprenant
c) une autre pluralité (N') d'au moins deux paires d'électrodes, chaque électrode étant adaptée à une application sur un segment de surface de ladite région de tissu biologique ou à une implantation dans celle-ci;
d) pour chaque paire d'électrodes de ladite autre pluralité, une autre source d'excitation électrique respective pour générer un autre champ électrique respectif entre les électrodes de ladite paire,
dans lequel chaque autre source d'excitation électrique est configurée pour générer un champ électrique sinusoïdal dépendant du temps, ayant une autre fréquence porteuse respective et une autre amplitude respective,
ainsi un autre champ électrique résultant dépendant du temps est formé dans une région où lesdits autre champs électriques respectifs se superposent spatialement,
l'appareil comprenant des autres moyens de commande permettant de faire fonctionner toutes desdites autre sources d'excitation électrique de telle sorte que:
- toutes lesdites autre fréquences porteuse respectives sont identiques à une autre fréquence porteuse présélectionnée (f₀'), l'autre fréquence porteuse étant choisie entre 500 Hz et 1 MHz,
- chacun desdits autre champs électriques sinusoïdaux dépendant du temps respectifs présente une autre différence de phase normalisée dépendant du temps définie, pour un cycle (φᵢ'(t), avec i=1 to N') par rapport à une phase de référence (φ₀' = 0), avec ${φ}_{i} ′ \left(t\right) = \left(i-1\right) ⋅ φ ′ \left(t\right)$
- et, si la fréquence de modulation de phase souhaitée est fₘ, alors l'autre différence de phase dépendant du temps est donnée par ${φ}_{i} ′ \left(t\right) = \left(i-1\right) ⋅ φ ′ \left(t\right) ⋅ f {′}_{m}$
ladite fréquence porteuse (f₀) et ladite autre fréquence porteuse (f₀') étant séparées par une différence de fréquence (Δf) d'au moins 500 Hz.

18. L'appareil selon la revendication 17, configuré de telle manière que la fréquence porteuse (f₀) et l'autre fréquence porteuse (f₀') soient modulées en phase de telle manière que la superposition par interférence de leurs champs électriques respectifs présente la même forme d'enveloppe et la même fréquence.

19. L'appareil selon la revendication 17, configuré de telle manière que la fréquence porteuse (f₀) et l'autre fréquence porteuse (f₀') soient modulées en phase de telle manière que la superposition par interférence de leurs champs électriques respectifs soit différente.

20. L'appareil selon la revendication 19, configuré de telle manière que les enveloppes d'excitation de deux régions différentes de la région du tissu biologique soient verrouillées en phase indépendamment par rapport à deux flux d'impulsions de synchronisation externes.
